Europäisches Patentamt
European Patent Office
Office européen des brevets

(19) (11) Veröffentlichungsnummer: **0 221 368 A2**

(12) **EUROPÄISCHE PATENTANMELDUNG**

(21) Anmeldenummer: **86113765.1**

(22) Anmeldetag: **04.10.86**

(51) Int. Cl.4: **C07C 125/067**, C07C 43/225, C07C 69/63, A61K 31/27, A61K 31/085, A61K 31/22

(30) Priorität: **31.10.85 DE 3538734**

(43) Veröffentlichungstag der Anmeldung: **13.05.87 Patentblatt 87/20**

(84) Benannte Vertragsstaaten: **AT BE CH DE ES FR GB GR IT LI LU NL SE**

(71) Anmelder: **Asta-Werke Aktiengesellschaft Chemische Fabrik**
**Artur-Ladebeck-Strasse 128 - 152 Postfach 14 01 29**
**D-4800 Bielefeld 14(DE)**

(72) Erfinder: **Schönenberger, Helmut, Prof. Dr.**
**Ahornstrasse 14**
**D-8401 Pentling(DE)**
Erfinder: **Schneider, Martin, Dr.**
**Steinmetzstrasse 11**
**D-8400 Regensburg(DE)**
Erfinder: **Schuderer, Michael, Dipl.-Chem.**
**Zollerstrasse 13**
**D-8400 Regensburg(DE)**
Erfinder: **Engel, Jürgen, Dr.**
**Erlenweg 3**
**D-8755 Alzenau(DE)**

(54) **Substituierte 1,1,2-Triphenylbut-1-ene.**

(57) Tumorhemmende 1,1,2-Triphenylbut-1-ene der allgemeinen Formel

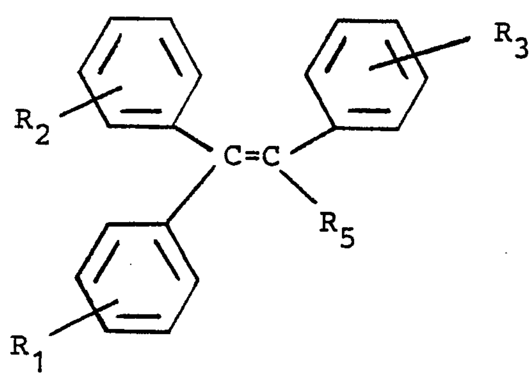

I

worin die Reste $R_1$, $R_2$ und $R_3$ gleich oder verschieden sind und Wasserstoff oder die Gruppe $OR_4$ bedeuten, und $R_4$ eine $C_3$-$C_6$-Alkenoylgruppe, eine Carbamoylgruppe, eine Carbamoylgruppe, die einen oder zwei β-Chlorethylreste oder β-Bromethylreste enthält, oder eine $C_2$-$C_6$-Alkanoylgruppe, welche ein Halogenatom enthält, bedeutet

EP 0 221 368 A2

und $R_5$ eine $C_1$-$C_6$-Alkylgruppe darstellt, wobei mindestens einer der Reste $R_1$, $R_2$ oder $R_3$ kein Wasserstoffatom ist, und worin einer oder zwei der Reste $R_1$, $R_2$ und $R_3$ auch eine Hydroxygruppe, die Gruppe $-OPO(OH)_2$, eine $C_2$-$C_6$-Alkanoyloxygruppe, welche gegebenenfalls eine Carboxygruppe enthält, oder eine 2-(Di-$C_1$-$C_4$-alkylamino)-ethyloxygruppe sein können, falls einer oder zwei dieser Reste die Carbamoyloxygruppe, oder eine Carbamoyloxygruppe, die einen oder zwei $\beta$-Chlorethylreste oder $\beta$-Bromethylreste enthält, darstellen und Verfahren zu deren Herstellung.

## Substituierte 1,1,2-Triphenylbut-1-ene

In J.Med.Chem. 25, 1070 (1982) werden acetoxysubstituierte 1,1,2-Triphenylbut-1-ene als mammatumorhemmend beschrieben.

Die Erfindung betrifft die durch die Patentansprüche definierten Gegenstände.

Die erfindungsgemäßen Verbindung besitzen eine Affinität zum Östrogenrezeptor und zeigen eine ausgeprägte verbesserte tumorhemmende Wirkung beispielsweise am hormonabhängigen MXT-Mammatumor der Maus. Die erfindungsgemäßen Verbindungen, insbesondere solche, die eine Carbamoyloxygruppe enthalten, werden vermehrt in der Mammakarzinomzelle angereichert und führen daher zu einer erhöhten östrogenantagonisierenden Wirkung und einer gesteigerten Hemmung des Wachstums des hormonabhängigen Mammakarzinoms.

Die erfindungsgemäßen Verbindungen besitzen auch eine antiandrogene Wirkung (zum Beispiel an der männlichen Maus (Züchtung NMRI); das Wachstum der Samenblase beziehungsweise der Prostata wird gegenüber unbehandelten Mäusen signifikant gehemmt). Dieser Effekt ist dem Effekt des als Arzneimittel verwendeten Östrogens Diethylstilböstrol vergleichbar, das zum Beispiel in der Therapie des Prostatakarzinoms Anwendung findet.

Weiterhin sind solche Verbindungen der Formel I, die beispielsweise eine durch β-Chlorethyl-oder β-Bromethyl substituierte Carbamoyloxygruppe, eine $C_3$-$C_6$-Alkenoyloxygruppe oder eine β-Chlor-beziehungsweise β-Brompropanoyloxygruppe ($-O-CO-CH_2-CH_2Cl$, $-O-CO-CH_2-CH_2Br$) enthalten, zusätzlich cytotoxisch wirksam und stellen daher östrogenrezeptoraffine Cytostatica dar; sie sind daher beispielsweise sowohl an hormonunabhängigen als auch hormonabhängigen Tumoren wirksam, wie zum Beispiel am Mammakarzinom (zum Beispiel hormonunabhängige menschliche MDA-Mammakarzinomzellinie; hormonabhängige menschliche MCF-7-Mammakarzinomzellinie; hormonabhängiger MXT-Mammatumor/Maus). Bei diesen Verbindungen erfolgt eine Anreicherung der cytotoxischen Wirkgruppe (zum Beispiel $Cl-CH_2-CH_2$, $Br-CH_2-CH_2$) in der Tumorzelle mit hoher Spezifität, was außerdem auch die systemische Toxizität des Cytostatikums vermindert. Da solche Verbindungen zwei verschiedenartige Angriffspunkte besitzen, das heißt neben einer östrogenantagonisierenden noch eine cytotoxische Wirkkomponente vorhanden ist, ist die Verwendung eines solchen östrogenrezeptoraffinen Cytostatikums vor allem dann von Vorteil, wenn bei der hormonellen Therapie des menschlichen Mammkarzinoms ein niedriger Rezeptorstatus beziehungsweise das Auftreten von Resistenz den Therapieerfolg in Frage stellen.

Insbesondere zeigen die erfindungsgemäßen Verbindungen gegenüber den vorbekannten Verbindungen bei der peroralen Langzeitanwendung eine überraschend bessere Wirkung.

Weiterhin zeichnen sich die erfindungsgemäßen Verbindungen durch eine gute Resorption, insbesondere im Magen-Darm-Trakt, und gute Verträglichkeit aus.

Falls $R_4$ eine $C_2$-$C_6$-Alkanoylgruppe ist, welche eine Carboxygruppe enthält, befindet sich eine solche Carboxygruppe insbesondere in β-oder ω-Stellung des Alkanoylrestes, wobei letzterer vorzugsweise 3 bis 5 Kohlenstoffatome enthält. Beispiele für derartige Gruppen sind: -Carboxypropionyl ($-CO-CH_2-CH_2-CO_2H$).

Falls $R_4$ eine $C_2$-$C_6$-Alkanoylgruppe ist, welche ein Halogenatom (vorzugsweise Chlor oder Brom) enthält, befindet sich dieses vorzugsweise in β-Stellung des Alkanoylrestes; Beispiele hierfür sind: $-CO-CH_2-CH_2Cl$, $-CO-CH_2-CH_2Br$.

Falls einer der Reste $R_1$, $R_2$ oder $R_3$ eine $C_2$-$C_6$-Alkanoyloxygruppe ist, handelt es sich vorzugsweise um eine $C_2$-$C_4$-Alkanoyloxygruppe, insbesondere die Acetoxygruppe. Als $C_3$-$C_6$-Alkenoyloxygruppe (das heißt $R_4$ = $C_3$-$C_6$-Alkenoyl) kommt beispielsweise die Gruppe $-O-CO-CH=CH_2$ in Frage. Die Substituenten $R_1$, $R_2$ und $R_3$ befinden sich vorzugsweise in der 4-Stellung der betreffenden Phenylringe. $R_5$ ist vorzugsweise eine Ethylgruppe.

Insbesondere handelt es sich bei den erfindungsgemäßen Wirkstoffen um folgende Verbindungen:

a) Verbindungen der Formel I, worin

$R_1$, $R_2$ und $R_3$ gleich sind und eine Carbamoyloxygruppe oder eine $C_3$-$C_6$-Alkenoyloxygruppe (vorzugsweise $-OCO-CH=CH_2$) oder eine $C_3$-$C_6$-Alkanoyloxygruppe, die ein Chlor-oder Bromatom in β-Stellung enthält - (vorzugsweise Chlor-oder Brompropionoxy), wobei einer oder zwei der Reste $R_1$, $R_2$ und $R_3$ auch Wasserstoff sein können (insbesondere ist $R_3$ Wasserstoff). Die Reste $R_1$, $R_2$ und $R_3$ befinden sich dabei vorzugsweise in 4-Stellung der Phenylringe. $R_5$ ist vorzugsweise die Ethylgruppe. Je nach Stellung und Zahl der Substituenten $R_1$, $R_2$ und $R_3$ liegen Östrogene, partielle oder echte Antiöstrogene vor. Die Verbindungen sind zum Beispiel am Mamma-und Prostatacarcinom wirksam. Wichtig ist, daß diese Verbindungen Östrogenrezeptor-affin sind, obwohl keine freien Hydroxygruppen vorhanden sind.

b) Verbindungen der Formel I, worin

$R_2$ eine Carbamoyloxygruppe ist, die gegebenenfalls einen oder zwei β-Chlorethyl-oder β-Bromethylreste enthält, $R_1$ Hydroxy, die Gruppe -O-PO(OH)$_2$, eine Carbamoyloxygruppe, eine 2-Dimethylaminoethyloxygruppe, eine $C_3$-$C_6$-Alkenoyloxygruppe oder eine $C_2$-$C_6$-Alkanoyloxygruppe darstellt, die gegebenenfalls in β-Stellung eine Carboxygruppe oder ein Halogenatom enthält und $R_3$ Wasserstoff ist oder die gleichen Bedeutungen wie $R_1$ hat, wobei im letzteren Falle auch $R_1$ Wasserstoff sein kann, oder worin $R_1$ eine Carbamoyloxygruppe ist, die gegebenenfalls einen oder zwei β-Chlorethyl-oder β-Bromethylreste enthält, $R_2$ Hydroxy, die Gruppe -O-PO(OH)$_2$, eine Carbamoyloxygruppe, eine 2-Dimethylethylamino-ethyloxygruppe, eine $C_3$-$C_6$-Alkenoyloxygruppe oder eine $C_2$-$C_6$-Alkanoyloxygruppe darstellt, die gegebenenfalls in β-Stellung eine Carboxygruppe oder ein Halogenatom enthält und $R_3$ Wasserstoff ist oder die gleichen Bedeutungen wie $R_2$ hat, wobei im letzteren Falle auch $R_2$ Wasserstoff sein kann. Die Substituenten $R_1$, $R_2$, $R_3$ befinden sich dabei vorzugsweise in 4-Stellung der Phenylringe.

$R_5$ ist vorzugsweise Ethyl.

c) Verbindungen der Formel I, worin

$R_2$ eine 2-(Di-$C_1$-$C_4$-alkylamino)-ethyloxygruppe (zum Beispiel Dimethylamino-ethyloxygruppe), vorzugsweise in 4-Stellung ist, $R_1$ eine Carbamoyloxygruppe ist (vorzugsweise in 4-Stellung), die gegebenenfalls auch einen oder zwei β-Chlor-oder β-Bromethylreste enthalten kann, und $R_3$ Wasserstoff bedeutet.

$R_5$ ist vorzugsweise Ethyl.

Die Einführung des Restes $R_4$ durch Acylierung erfolgt mittels einer Säure der Formel R-OH, wobei R eine Aminocarbonylgruppe, eine Aminocarbonylgruppe, die einen oder zwei β-Chlorethylreste oder β-Bromethylrest am N-Atom enthält, die Gruppe -PO(OH)$_2$, eine $C_3$-$C_6$-Alkenoylgruppe, eine $C_2$-$C_6$-Alkanoylgruppe oder eine $C_2$-$C_6$-Alkanoylgruppe, die eine zusätzliche Carboxygruppe oder ein Halogenatom - (insbesondere Chlor oder Brom und zwar in β-Stellung) enthält, darstellt und eine solche Säure vorzugsweise aktiviert ist. Falls für die Acylierung eine solche aktivierte Säure verwendet wird, handelt es sich vorzugsweise um Verbindungen der Formel R-X, worin R die oben angegebenen Bedeutungen hat und X ein Halogenatom, eine Gruppe der Formel -OR', -SR' oder eine Gruppe der Formel -$OSO_3H$ oder -OCO-R" bedeutet und R' einen $C_1$-$C_6$-Alkylrest oder im Falle von -OR' beziehungsweise -SR' auch einen Phenylrest, einen durch Nitrogruppen, $C_1$-$C_4$-Alkoxygruppen, $C_1$-$C_4$-Alkylgruppen oder Halogenatome (Chlor, Fluor, Brom) substituierten Phenylrest, einen Cyanomethylrest oder einen Carboxymethylrest und R" einen geraden oder verzweigten $C_1$-$C_5$-Alkylrest oder $C_2$-$C_5$-Alkenylrest, einen $C_1$-$C_5$-Alkylrest mit einer zusätzlichen Carboxygruppe oder einem zusätzlichen Halogenatom darstellt, wobei R auch die Gruppe $POCl_2$ oder die Cyangruppe sein kann, falls X Halogen ist und sich in diesen Fällen noch eine Verseifung mittels einer Mineralsäure (Schwefelsäure, Salzsäure) anschließt, oder wobei R auch die Gruppe -COCl sein kann, falls X Halogen ist und in diesem Falle das erhaltene Reaktionsprodukt noch mit Ammoniak oder β-Chlorethylamin, β-Bromethylamin, Bis-β-Chlorethylamin oder Bis-β-bromethylamin umgesetzt wird. Gegebenenfalls wird hierbei ein säurebindender Stoff (zum Beispiel tertiäre aliphatische Amine, Pyridin) zugesetzt.

Die Verseifung mit Mineralsäuren erfolgt zum Beispiel in wässrigem Medium bei Temperaturen zwischen 50 und 100° C. Die Umsetzung mit Ammoniak oder den zuvor angegebenen β-Chlorethyl-beziehungsweise β-Bromethylaminen findet in inerten Lösungsmitteln statt, die für die Acylierung in Frage kommen, wobei der Temperaturbereich zum Beispiel zwischen 0 und 25° C liegt.

Falls X ein Halogenatom bedeutet, handelt es sich vorzugsweise um Chlor, Brom oder Jod; falls R' beziehungsweise R" Alkylreste oder Alkoxyreste bedeuten, dann sind diese vorzugsweise niedermolekular und bestehen aus 1 bis 4 Kohlenstoffatomen.

Die Acylierung wird beispielsweise in einem üblichen Lösungsmittel oder Suspensionsmittel wie Wasser, gegebenenfalls unter Zusatz eines Lösungsvermittlers (zum Beispiel niedere aliphatische Alkohole, niedere aliphatische Ketone, Dimethylformamid) oder indifferenten Mitteln durchgeführt. Als Lösungs-beziehungsweise Suspensionsmittel kommen beispielsweise in Betracht: niedere aliphatische Alkohole mit 1 -6 C-Atomen (zum Beispiel n-Butanol, tert.-Butanol), niedrigmolekulare aliphatische Ether (zum Beispiel 4-10 C-Atome); niedrigmolekulare aliphatische Ketone (zum Beispiel 3-6 C-Atome); gesättigte cyclische Ether, wie Tetrahydrofuran, Dioxan, niedrigmolekulare gesättigte Chlor-und Fluorkohlenwasserstoffe mit 1-5 C-Atomen, wobei die einzelnen C-Atome ein-oder mehrfach (2-bis 3-fach) durch Chlor und/oder Fluor substituiert sein können, wie Chloroform, Methylenchlorid; gegebenenfalls durch Chlor oder Brom substituierte aromatische Kohlenwasserstoffe wie Benzol, Toluol, Xylol, Chlorbenzol, Dimethylformamid, Dimethylsulfoxid, Tetramethylharnstoff, Pyridin, N-Methylpyrrolidon. Es können selbstverständlich auch Mischungen dieser genannten Lösungsmittel verwendet werden.

Das Verfahren wird bei Temperaturen zwischen 0 und 200° C vorzugsweise 15 und 150° C durchgeführt.

Häufig, insbesondere wenn X ein Halogenatom oder die Gruppe -OCOR" bedeutet, ist die Gegenwart eines säurebindenden Stoffes wie Alkalihydroxiden, Alkalicarbonaten, Alkalihydrogencarbonaten, Alkaliacetaten, Erdalkalicarbonaten, Trialkylaminen, Dialkylaminen, Pyridin und ähnlichen zweckmäßig. Dabei kann das säurebindende Agens auch gleichzeitig allein oder im Gemisch mit anderen üblichen Mittels als Lösungsmittel benutzt werden (zum Beispiel Pyridin).

Bei der Acylierung kann man auch so vorgehen, daß man erst von der umzusetzenden Verbindung eine Alkaliverbindung herstellt, indem man sie in einem inerten Lösungsmittel wie Dioxan, Dimethylformamid, Benzol oder Toluol mit einem Alkalimetall, Alkalihydriden oder Alkaliamiden (insbesondere Natrium oder Natriumverbindungen) bei Temperaturen zwischen 0 und 150° C umsetzt und zum Beispiel dann das acylierende Agens (Verbindung R-X, X = Halogen) zufügt.

Falls die freie Säure R-OH verwendet wird, so ist deren Aktivierung durch die Gegenwart von Kondensationsmitteln wie Dicyclohexylcarbodiimid, Tetraäthylpyrophophit, 5-(3'-Sulfonphenyl)-äthylisooxazol, Schwefligsäure-bis-alkylamiden (zum Beispiel $SO[N(CH_3)_2]_2$), N,N'-Carbonyldiimidazol und so weiter erforderlich (Organic Reactions, Vol. 12, 1962, Seiten 205 und 239).

Bei der Acylierung (Einführung von $R_4$ beziehungsweise einer $C_2$-$C_6$-Alkanoylgruppe) kann man auch selektiv vorgehen, das heißt man acyliert zum Beispiel nur eine oder zwei der freien Hydroxygruppen der Ausgangssubstanz II; dies erreicht man beispielsweise durch entsprechende Acylierung einer Ausgangssubstanz II, worin $R_3$ Wasserstoff ist und die Reste $R_1$ und $R_2$ Hydroxygruppen sind (vorzugsweise in gleichen Positionen des Phenylringes) mit nur einem Mol des Acylierungsmittels pro 1 Mol der Verbindung II. In der so erhaltenen Verbindung kann die freie Hydroxygruppe wie vorstehend beschrieben auch durch einen anderen Acylrest $R_4$ acyliert werden.

Eine andere Möglichkeit der selektiven Acylierung besteht darin, daß man von einer Ausgangsverbindung der Formel II ausgeht, worin einer oder zwei der Reste $R_1$, $R_2$ oder $R_3$ verethert sind (das heißt eine $C_1$-$C_6$-Alkoxygruppe darstellen), die anderen Hydroxy oder Wasserstoff bedeuten (einer der Reste $R_1$, $R_2$ oder $R_3$ jedoch mindestens eine Hydroxygruppe darstellt), und die vorliegenden freien Hydroxygruppen in der angegebenen Weise acyliert und anschließend die vorhandenen Alkoxygruppen in bekannter Weise abspaltet. Auch hier können die so erhaltenen freien Hydroxygruppen in der angegebenen Weise anschließend zusätzlich durch einen Acylrest $R_4$ acyliert werden.

Die Abspaltung von Ethergruppen erfolgt beispielsweise ohne Lösungsmittel oder in einem inerten Lösungsmittel mit Bortribromid, Bortrifluorid, Aluminiumchlorid, Siliziumtetrachlorid, Aluminiumtribromid, Natriumethylthiolat, $(CH_3)_3SiCl + NaJ$ bei Temperaturen zwischen -70° C und 200° C. Als Lösungsmittel für diese Etherspaltung kommen beispielsweise in Frage: aliphatische Halogenkohlenwasserstoffe wie zum Beispiel Methylenchlorid, aromatische Kohlenwasserstoffe wie Benzol, Toluol, Xylol, halogenierte aromatische Kohlenwasserstoffe wie Chlorbenzol, Dichlorbenzole, Dimethylformamid, Acetonitril.

Weiterhin kann diese Etherspaltung auch mittels konzentrierter Jod-Wasserstoffsäure, Pyridinhydrochlorid, Bromwasserstoffsäure, Methylmagnesiumjodid mit oder ohne Lösungsmittel bei Temperaturen zwischen 20° C bis 250° C erfolgen. Als Lösungsmittel für die zuletzt genannte Spaltung kommen beispielsweise in Betracht: aliphatische Ether mit Alkylresten aus 1 bis 6 C-Atomen.

Die Acylgruppen in den Verbindungen der Formel I können solvolytisch wieder abgespalten werden, wodurch die entsprechenden freien Hydroxy-Verbindungen der Formel I erhalten werden. Diese solvolytische Abspaltung erfolgt beispielsweise durch Verseifung mit verdünnten Säuren oder mittels basischer Substanzen (Pottasche, Soda, wässrige Alkalilösungen, alkoholische Alkalilösungen, $NH_3$) bei Temperaturen zwischen 10 und 150° C, insbesondere 20 bis 100° C.

Es ist auch eine selektive Abspaltung möglich, indem nur ein Acylrest ($R_4$) abgespalten wird.

Verbindungen der Formel I, worin einer oder mehrere der Reste $R_1$, $R_2$ und $R_3$ eine $C_3$-$C_6$-Alkenoyloxygruppe haben, können auch aus entsprechenden Verbindungen I erhalten werden, bei denen einer oder mehrere der Reste eine $C_3$-$C_6$-Alkanoyloxygruppe darstellen, die in $\beta$-Stellung ein Chlor-oder Bromatom enthalten, indem man mittels einer Base (zum Beispiel einem tertiären Amin, wie Triethylamin, Tripropylamin, N-Methylpyrrolidin und Pyridin) das Halogenatom unter Ausbildung einer $\alpha$-$\beta$-ständigen Doppelbindung abspaltet. Diese Reaktion findet zum Beispiel bei Temperaturen zwischen 100 und 150° C in einem inerten Lösungsmittel (aromatische Kohlenwasserstoffe, wie Toluol, Xylol und Chlorbenzol oder Halogenwasserstoff, wie Methylenchlorid oder Chloroform) statt.

Wenn die beiden linken Phenylreste in der Formel I verschieden sind, erhält man die erfindungsgemäßen Verbindungen der Formel I im allgemeinen in Form von Gemischen der entsprechenden cis-und trans-Isomeren. Dabei wird unter der cis-Forme eine Verbindung I verstanden, wo der Phenylring mit dem Substituenten $R_2$ in cis-Stellung zu dem Phenylring mit dem Substituenten $R_3$ steht. Steht der Phenylring mit dem Substituenten $R_2$ in trans-Stellung zu dem Phenylring mit dem Substituenten $R_3$, dann liegt die trans-Form vor.

Die Trennung der cis-und trans-Isomeren kann mittels beliebiger dem Fachmann bekannter Verfahren zur Trennung von cis-und trans-Isomeren ausgeführt werden, zum Beispiel durch fraktionierte Kristallisation der gemischten Isomeren oder der Salze derselben aus einem organischen Lösungsmittel oder Gemisch von Lösungsmitteln, zum Beispiel Methanol, Aceton oder Petrolether oder ein Gemisch derselben, oder durch chromatographische Trennung der gemischten Isomeren oder der Salze derselben.
Besonders wirksam sind im Falle des Vorkommens von Isomeren die cis-Formen.
Natürlich können die erfindungsgemäßen Verbindungen der Formel I auch in Form ihrer cis-trans-Gemische zur Anwendung kommen.

Falls die Verbindungen der Formel I eine Carboxygruppe enthalten, können solche Verbindungen auch in Form der entsprechenden Salze mit physiologisch verträglichen anorganischen oder organischen Kationen vorliegen. Als anorganische Kationen kommen zum Beispiel in Frage: $NH_4^+$, Kationen der Alkalimetalle (Na, K), der Erdalkalimetalle (Ca, Mg), des Wismuts, Aluminiums oder des Eisens.

Als organische Kationen kommen zum Beispiel in Frage: Kationen von primären, sekundären und tertiären $C_1$-$C_6$-Alkylaminen, die auch eine zweite Aminogruppe enthalten können (wie zum Beispiel Kationen des Trimethylamins, des Triethylamins oder des Ethylendiamins), von cyclischen Aminen - (Piperidin, Morpholin, Piperazin) oder von $C_4$-$C_8$-Cycloalkylaminen (zum Beispiel Cyclohexylamin).

Die Ausgangsstoffe sind bekannt beziehungsweise können ausgehend von entsprechenden 1-Methoxyphenyl-2-phenyl-ethanonen oder von 1-Methoxyphenyl-2-methoxyphenyl-ethanonen (das heißt die Methoxygruppen können an verschiedenen Stellen des Phenylringes sitzen) nach beziehungsweise analog dem von Dodds, Proc. Roy. Soc. B 127, 148 (1939) beschriebenen Verfahren hergestellt werden (vgl. hierzu auch Beispiel 1).
Das entsprechende 1,2-Diphenylethanon ist zum Beispiel durch Friedel-Crafts-Acylierung von Anisol durch das gegebenenfalls mit einer Methoxygruppe substituierte Phenylacetylchlorid zugänglich. Daran schließt sich die Ethylierung zum 1,2-Diphenylbutan-1-on an. Nach der Grignard-Addition mit Methoxyphenylmagnesiumbromid und anschließender Wasserabspaltung mittels eines $H_2SO_4$/Eisessig-Gemisches erhält man das Methoxysubstituierte 1,1,2-Triphenylbut-1-en.

Synthese des N,N-Bis-β-chlorethyl-chlorformamids: 21,0 g Bis-β-chlorethylaminhydrochlorid (1,2 Mol) werden in wenig Wasser gelöst und mit kalter $Na_2CO_3$-Lösung alkalisiert. Die ausgeschiedene Base wird in eiskaltem Ether aufgenommen -es wird noch zweimal mit eiskaltem Ether ausgeschüttelt. Die Etherphasen werden unter Kühlung über $Na_2SO_4$ getrocknet. Der Ether wird bei 5° C am Rotationsverdampfer abgezogen und die so isolierte Nor-Lost-Base in 400 ml trockenem Benzol aufgenommen. Unter Eiskühlung wird diese Lösung tropfenweise mit einer Lösung von 7,3 g (0,07 Mol) Phosgen in trockenem Toluol versetzt. Man läßt es einige Stunden stehen, filtriert das ausgeschiedene Bis-β-chlorethylaminhydrochlorid ab und entfernt das Lösungsmittel am Rotationsverdampfer. Der gelbe ölige Rückstand wird im Hochvakuum bei 1,5 Torr und 125° C destilliert.
Das entsprechende N,N-Bis-β-bromethyl-chlorformamid kann analog hierzu hergestellt werden.

Die erfindungsgemäßen Verbindungen zeigen am hormonabhängigen MXT-M 3.2 Mammatumor der Maus eine gute Wirkung; die Wirkung der β-Halocarbonsäureester ist besonders hervorstechend. Beispielsweise wird nach subcutaner Implantation des MXT 3.2 Mammatumors in die BDF 1-Maus bei einer Dosis von 1,2 mg/kg Körpergewicht/Maus ein %T/C-Wert von 1% erzielt. Der Buchstabe T bedeutet "behandelte Tiergruppe"; C bedeutet "nicht-behandelte Kontrollgruppe". Ein %T/C-Wert von 1% bedeutet dann, daß das Tumorgewicht der behandelten Gruppe nur 1% der Tumorgewichts der nicht-behandelten Kontrollgruppe ist.

Die niedrigste, bereits wirksame Dosis in dem obengenannten Tierversuch ist beispielsweise
6 mg/kg oral
2 mg/kg subcutan
2 mg/kg intravenös

Als allgemeiner Dosisbereich für die Wirkung (Tierversuch wie oben) kommt beispielsweise in Frage:
6 -30 mg/kg oral, insbesondere 15 mg
2 -10 mg/kg subcutan, insbesondere 6 mg
2 -10 mg/kg intravenös, insbesondere 6 mg

Die Wirkungsrichtung der erfindungsmäßen Verbindungen ist mit der Wirkung des bekannten Arzneimittels Tamoxifen vergleichbar, jedoch bestehen hierzu insbesondere folgende Unterscheide:
Die erfindungsgemäßen Verbindungen wirken auch an hormonunabhängigen Mammatumorzellen
Indikation für die die erfindungsgemäßen Verbindungen in Betracht kommen können: hormonabhängige Tumore wie Endometrium-, Ovarial-, Prostata-, Cervix-und Mammacarzinom
Kontraindikation: keine

Die pharmazeutischen Zubereitungen enthalten im allgemeinen zwischen 10 bis 200 vorzugsweise 10 bis 40 mg der erfindungsgemäßen aktiven Komponente(n).

Die Verabreichung kann beispielsweise in Form von Tabletten, Kapseln, Pillen, Dragees, Zäpfchen, Salben, Gelees, Cremes oder in flüssiger Form erfolgen. Als flüssige Anwendungsform kommen zum Beispiel in Frage: Ölige oder alkoholische beziehungsweise wässrige Lösungen sowie Suspensionen und Emulsionen.

Bevorzugte Anwendungsformen sind Tabletten, die zwischen 10 mg und 40 mg oder Lösungen, die zwischen 0,01 bis 1 % an aktiver Substanz enthalten.

Die Einzeldosis der erfindungsgemäßen aktiven Komponenten kann beispielsweise liegen

a) bei oralen Arzneiformen zwischen 10 und 40 mg
vorzugsweise 15 mg

b) bei parenteralen Arzneiformen (zum Beispiel intravenös, intramuskulär) zwischen 2 -10 mg
vorzugsweise 6 mg

c) bei Arzneiformen zur rektalen oder vaginalen Applikation zwischen 10 und 40 mg
vorzugsweise 15 mg

d) bei Arzneiformen zur lokalen Applikation auf die Haut und Schleimhäute (zum Beispiel in Form von Lösungen, Lotionen, Emulsionen, Salben und so weiter) zwischen 2 und 10 mg
vorzugsweise 6 mg

-(Die Dosen sind jeweils bezogen auf die freie Base) -

Beispielsweise können 3 mal täglich 1 bis 4 Tabletten mit einem Gehalt von 10 bis 40 mg wirksamer Substanz oder zum Beispiel bei intravenöser Injektion 1 bis 4 mal täglich eine Ampulle von 10 bis 25 ml Inhalt mit 2 bis 10 mg Substanz empfohlen werden. Bei oraler Verabreichung ist die minimale tägliche Dosis beispielsweise 10; die maximale tägliche Dosis bei oraler Verabreichung soll nicht über 200 liegen.

Die akute Toxizität der erfindungsgemäßen Verbindungen an der Maus (ausgedrückt durch die LD 50 mg/kg; Methode nach Miller und Tainter: Proc. Soc. Exper. Biol. a. Med. 57 (1944) 261) liegt beispielsweise bei oraler Applikation zwischen 1000 und 5000 mg/kg (beziehungsweise oberhalb 1000 mg/kg.

Beispiel 1

1,1-Bis-(4-carbamoyloxy-phenyl)-2-phenyl-but-1-en

Zu einer Lösung von 25 g (25 mMol) Phosgen in wasserfreiem Toluol gibt man unter Rühren und Kühlung eine Suspension aus 10 mMol 1,1-Bis-(4-hydroxy-phenyl)-2-phenyl-but-1-en und 2,5 g (20 mMol) Dimethylanilin in wasserfreiem Toluol. Die Reaktionssuspension wird weitere 12 Stunden bei Raumtemperatur gerührt und anschließend mit verdünnter HCl, gesättigter $NaHCO_3$-Lösung und $H_2O$ gewaschen, dann über $Na_2SO_4$ getrocknet. In die Toluollösung wird $NH_3$ eingeleitet. Das 1,1-Bis-(4-carbamoyloxy-phenyl)-2-phenyl-but-1-en fällt aus.
F. 202° C
Ausbeute: 65 % der Theorie

Die Ausgangssubstanz kann zum Beispiel wie folgt erhalten werden:
Zu einer Lösung von 15,5 g (0,1 Mol) Phenylacetylchlorid in 80 ml Dichlorethan werden 21,6 g (0,2 Mol) Anisol und darauf unter Rühren und Eiskühlung noch 24,0 g (0,2 Mol) $AlCl_3$ portionsweise zugegeben. Es wird 5 Stunden unter Rückfluß erhitzt und für weitere 2 Stunden bei Raumtemperatur gerührt. Die Mischung wird unter Rühren auf 500 ml Eiswasser gegossen und die Dichlorethanphase abgetrennt. Die wäßrige Phase wird zweimal mit $CHCl_3$ extrahiert. Die vereinigten organischen Phasen werden zweimal mit 10 %iger Natronlauge und dreimal mit Wasser gewaschen und über $CaCl_2$ getrocknet. Das Lösungsmittel wird am Rotationsverdampfer abgezogen. Das so erhaltene 1-(4-Methoxy-phenyl)-2-phenyl-ethanon schmilzt bei 77° C.

Eine noch heiße Lösung von 2,3 g (0,1 Grammatome) Natrium in 44 ml absolutem Ethanol werden zu einer Mischung von 22,6 g (0,1 Mol) 1-(4-Methoxy-phenyl)-2-phenyl-ethanon und 15,6 g (0,1 Mol) Ethyljodid unter Rühren zugetropft. Nach Abklingen der heftigen Reaktion wird 15 Minuten unter Rückfluß erhitzt. Es wird noch zweimal in Abständen von je 30 Minuten eine Lösung von 1,2 g (0,05 Grammatome) Natrium in 22 ml absolutem Ethanol und 7,8 g (0,05 Mol) Ethyljodid zugegeben und 4 Stunden unter Rückfluß erhitzt. Ethanol und überschüssiges Ethyljodid werden am Rotationsverdampfer abgezogen. Nach Zugabe von $H_2O$

zum Rückstand wird zweimal mit Ether ausgeschüttelt. Die organischen Phasen werden mit $Na_2S_2O_3$-Lösung und $H_2O$ gewaschen. Das Lösungsmittel wird nach Trocknen über $CaCl_2$ abgezogen und das erhaltene 1-(4-Methoxy-phenyl)-2-phenylbutan-1-on umkristallisiert.
F.45° C.

7,3 g (0,3 Grammatome) Magnesium werden mit 10 ml absolutem Ether überschichtet. Es wird eine Lösung von 56,1 g (0,3 Mol) 4-Brom-Anisol in 200 ml Ether zugetropft. Nach Abklingen der Reaktion erhitzt man noch 30 Minuten unter Rückfluß. Es werden nun 0,1 Mol = 25,4 g (0,1 Mol) 1-(4-Methoxy-phenyl)-2-phenyl-butan-1-on in 60 ml absolutem Ether zur Grignardlösung getropft. Nach Abschluß des Zutropfens wird 2 Stunden unter Rückfluß erhitzt. Nach Hydrolyse mit Eiswasser/3N $H_2SO_4$ wird die wäßrige Phase mit Ether ausgeschüttelt. Die Etherphasen werden mit gesättigter $NaHCO_3$-Lösung und $H_2O$ gewaschen, über $CaCl_2$ getrocknet und mit Ether abgezogen. Zum Rohprodukt der Grignard-Reaktion werden nun 50 ml einer $H_2SO_4$/Eisessig-Mischung (Volumenverhältnis 2:8) zugegeben. Darauf wird 20 Minuten bei 70° C auf dem Wasserbad erhitzt. Nach Abkühlen und Zugabe von Wasser wird zweimal mit Ether extrahiert. Die Etherphasen werden mit NaOH und $H_2O$ gewaschen und über $CaCl_2$ getrocknet. Der Ether wird abgezogen und das Rohprodukt umkristallisiert. Das so erhaltene 1,1-Bis-(4-methoxy-phenyl)-2-phenyl-but-1-en - schmilzt bei 115° C.

Eine Lösung von 3,5 g 1,1-Bis-(4-methoxy-phenyl)-2-phenyl-but-1-en (0,01 Mol) in 150 ml absolutem $CH_2Cl_2$ wird unter Stickstoff auf -60° C gekühlt. Unter Rühren werden 7,5 g $BBr_3$ (0,03 Mol) zugegeben. Nach 15 Minuten wird das Kühlbad entfernt und weitere 3 Stunden bei Raumtemperatur gerührt. Schließlich gibt man unter Kühlung noch 10 ml Methanol zu und läßt 30 Minuten rühren. Das Lösungsmittel wird bei 25° C im Vakuum abgezogen. Das Rohprodukt wird mit 2N NaOH versetzt und bei Raumtemperatur solange gerührt bis alles gelöst ist. Nach Ansäuern mit 3N $H_2SO_4$ fällt das 1,1-Bis-(4-hydroxy-phenyl)-2-phenyl-but-1-en aus. Es wird aus Methanol umkristallisiert.
F. 200° C; Ausbeute: 90 % der Theorie.

Beispiel 2

1,1-Bis-(4-β-chlor-propionoxy-phenyl)-2-phenyl-but-1-en

5 mmol = 1,6 g 1,1-Bis-(4-hydroxyphenyl)-2-phenyl-but-1-en werden in 50 mmol (=6,4 g) β-Cl-propionsäurechlorid suspendiert. Nach 2 Stunden Rühren bei 120° C wird das überschüssige Säurechlorid in Vakuum abgezogen. Der ölige Rückstand wird über eine Kieselgelsäule (Laufmittel: $CHCl_3$/Essigester 10 : 1) gereinigt und das Produkt aus Ethanol umkristallisiert.
Ausbeute: 81 %
F. 82° C.

Beispiel 3

1,1-Bis-(4-β-brom-propionoxy-phenyl)-2-phenyl-but-1-en

Herstellung erfolgt analog Beispiel 2, indem lediglich anstelle des β-Chlor-propionsäurechlorids nun 8,6 g β-Brom-propionsäurechlorid verwendet werden.
Ausbeute: 72 %
F. 94° C.

Beispiel 4a

1-[4-(N,N-Bis-β-chlorethylcarbamoyloxy)-phenyl]-1-(4-hydroxy-phenyl)-2-phenyl-but-1-en

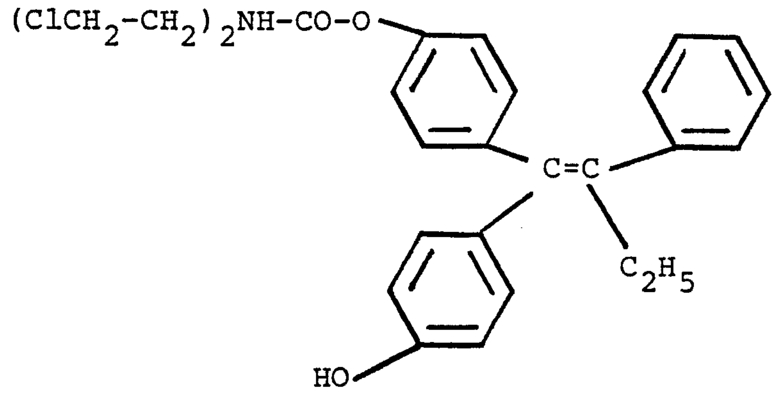

Zu einer Lösung von 6,4 g (0,02 Mol) 1,1-Bis-(4-hydroxy-phenyl)-2-phenyl-but-1-en in Pyridin gibt man 3,8 g (0,02 Mol) Chlorameisensäure-bis-β-chlorethylamid und läßt den Ansatz 4 Tage bei Raumtemperatur rühren. Das ausgefallene Pyridinhydrochlorid wird abfiltriert, das Pyridin abdestilliert. Das Produkt wird auf einer Kieselgelsäule (Laufmittel $CHCl_3$/Essigsäureethylester 10:1) isoliert und die Isomeren (cis-trans Isomere) mittels fraktionierter Kristallisation aus Ethanol getrennt.

Das Isomere der oben angegebenen Formel (der Carbamoylrest steht cis-ständig zur unsubstituierten Phenylgruppe) schmilzt bei 135° C. Ausbeute: 32 %.

Das andere Isomere (Carbamoylrest ist trans-ständig zur unsubstituierten Phenylgruppe) wurde nur als Gemisch mit der cis-Verbindung vom Schmelzpunkt 128°C -129°C isoliert.

Beispiel 4b

1-[4-N,N-Bis-β-bromethylcarbamoyloxy)-phenyl]-1-(4-hydroxyphenyl)-2-phenyl-but-1-en

Die Herstellung erfolgt analog Beispiel 4a unter Verwendung von Chlorameisensäure-bis-β-bromethylamid. Farblose Kristalle, F.: 125° C, Ausbeute: 28 %.

Beispiel 5

1-[4-(N,N-Bis-β-chlorethylcarbamoyloxy-phenyl]-1,2-bis-(4-hydroxy-phenyl)-but-1-en

3,6 g (0,01 Mol) 1,2-Bis-(4-methoxy-phenyl)-1-(4-Hydroxy-phenyl-but-1-en werden mit 7,7 g (0,04 Mol) Chlorameisensäure-bis-β-chlorethylamid in Pyridin gelöst und 4 Tage lang bei Raumtemperatur gerührt. Das Pyridin wird abrotiert und das Produkt mittels Säulenchromatographie (Kieselgel: $CHCL_3$/Essigsäureethylester 10:1) isoliert und in Ethanol umkristallisiert. Das erhaltene 1-[4-(N,N-Bis-β-chlorethylcarbamoyloxy)-phenyl]-1,2-bis-(4-methoxy-phenyl)-but-1-en schmilzt bei 73° C (Ausbeute: 42 % der Theorie).

Eine Lösung von 5,3 g (0,01 Mol) dieser Substanz in 150 ml absolutem $CH_2Cl_2$ wird unter Stickstoff auf -60° C gekühlt. Unter Rühren wird eine ebenfalls gekühlte Lösung von 7,5 g (0,03 Mol) Bortribromid in 50 ml absolutem $CH_2Cl_2$ langsam zugetropft. Nach 15 Minuten wird das Kühlbad entfernt, man läßt 3 Stunden bei Raumtemperatur rühren und gibt anschließend unter Kühlung 10 ml Methanol zu. Nach einer halben Stunde Rühren wäscht man den Ansatz mit gesättigter $NaHCO_3$-Lösung, trocknet die $CH_2Cl_2$-Phase über $NaSO_4$, destilliert das Lösungsmittel ab und isoliert das Produkt mittels Säulenchromatographie (Kieselgel; Laufmittel $CHCl_3$/Essigsäureethylester 2:1). Die so erhaltene entmethylierte Verbindung schmilzt bei 138° C. (cis-trans-Gemisch) Ausbeute: 35 % der Theorie.

Herstellung der Ausgangssubstanz:

7,3 g (0,3 Grammatom) Magnesium werden mit 10 ml absolutem Tetrahydrofuran überschichtet. Es wird eine Lösung von 77,2 g (0,3 Mol) 4-Brom-phenyl-tetrahydropyranylether in 200 ml absolutem Tetrahydrofuran zugetropft. Nach Abklingen der Reaktion erhitzt man noch 30 Minuten unter Rückfluß. Es werden nun 28,4 g (0,1 Mol) 1,2-Bis-(4-Methoxy-phenyl)-butan-1-on in 60 ml absolutem Tetrahydrofuran zur

Grignardlösung getropft. Nach Abschluß des Zutropfens wird 2 Stunden unter Rückfluß erhitzt. Der Ansatz wird in 500 ml Eiswasser/3N $H_2SO_4$ gegeben und die wäßrige Phase mit Ether ausgeschüttelt. Die Etherphasen werden mit gesättigter $NaHCO_3$-Lösung und $H_2O$ gewaschen über $Na_2SO_4$ getrocknet und der Ether abgezogen.

Zum Rohprodukt der Grignardreaktion gibt man nun 70 ml eines Methanol/3N HCl-Gemisches (Volumen 1:1) und läßt über Nacht bei Raumtemperatur rühren. Das Methanol wird abdestilliert, der Rückstand in Wasser aufgenommen und mit Ether ausgeschüttelt. Die Etherphasen werden mit $NaHCO_3$-Lösung und Wasser gewaschen. Das Produkt mittels Säulenchromatographie (Kieselgel; Laufmittel: $CHCL_3$/Essigsäureethylester 10:1) isoliert und in Ethanol zur Kristallisation gebracht. Das so erhaltene 1,2-Bis-(4-methoxy-phenyl)-1-(4-hydroxy-phenyl)-but-1-en schmilzt bei 82° C.

Beispiel 6

1-[4-(N,N-Bis-β-chlorethylcarbamoyloxy)-phenyl]-1-(4-hydroxyphenyl)-2-(3-hydroxyphenyl)-but-1-en

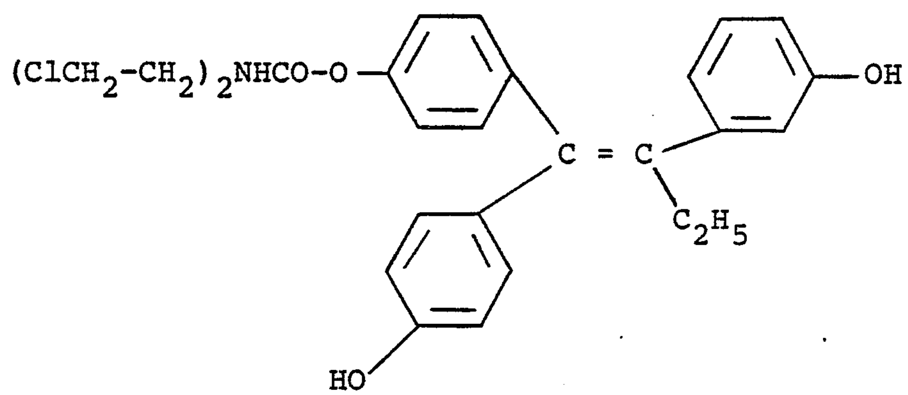

Die Synthese erfolgt analog Beispiel 5. Man geht lediglich vom 1-(4-Hydroxy-phenyl-1-(4-methoxyphenyl)-2-(3-methoxyphenyl)-but-1-en anstelle des 1,2-Bis-(4-methoxyphenyl)-1-(4-hydroxy-phenyl-buten-1-en aus. Ausbeute: 43 %

F. 92-94° C (cis-trans-Gemisch)

Die Herstellung der Ausgangssubstanz erfolgt analog Beispiel 5.

Beispiel 7

1-[4-(N,N-Bis-β-chlorethylcarbamoyloxy)-phenyl]1,2-bis-(4-hydroxyphenyl)-but-1-en

0,5 g (≙ 1 mmol) 1-[4-(N,N-Bis-β-chlorethylcarbamoyloxy)-phenyl]-1,2-bis-(4-hydroxyphenyl)-but-1-en werden mit 1,5 g (≙ 6 mmol) Acetanhydrid in 4 ml Pyridin eine Stunde bei 80° C gerührt. Das Pyridin wird abdestilliert und der ölige Rückstand mittels Säulenchromatographie (Kieselgel: $CHCl_3$/Essigsäureethylester 10:1) gereinigt.

Das Produkt kritallisiert aus Ethanol.

Ausbeute: 53,0 %

F. 108° C.

Beispiel 8

1,1-Bis-(4-acroyloxy-phenyl)-2-phenyl-but-1-en

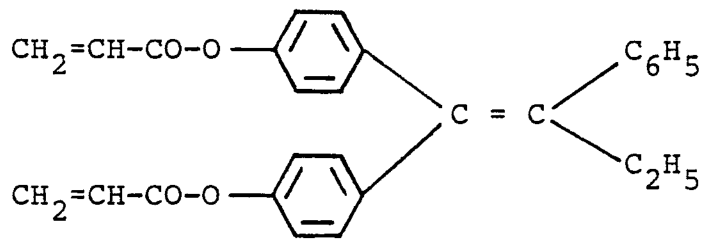

2,5 mmol (≙ 1,3 g) 1,1-Bis-(4-β-chlor-propionoxy-phenyl)-2-phenyl-but-1-en, 8 mmol (≙ 0,8 g) Triethylamin und 10 mg p-Hydrochinon werden in 50 ml trockenem Toluol gelöst und 20 Stunden unter Rückfluß erhitzt. Das Toluol wird abdestilliert, der ölige Rückstand über eine Kieselgelsäule (Laufmittel: $CHCl_3$/Essigester 10:1) gereinigt und das Produkt aus Ethanol umkristallisiert.
Ausbeute: 52,8 %
F. 79° C.

Beispiel 9

1,1-Bis-[4-(N,N-bis-β-chlorethylcarbamoyloxy)-phenyl]-2-phenyl)-but-1-en

Zu 8,0 g (0,04 mol) Chlorameisensäure-bis-β-chlorethylamid gibt man eine Lösung von 3,2 g (0,01 Mol) 1,1-Bis-(4-hydroxyphenyl)-2-phenyl-but-1-en in 50 ml Pyridin und läßt den Ansatz 4 Tage bei Raumtemperatur stehen. Das ausgefallene Pyridinhydrochlorid wird abfiltriert, das Pyridin im Vakuum abdestilliert. Der Rückstand wird über eine Kieselgelsäule gereinigt (Laufmittel: $CHCl_3$/Essigester 10:1) und in Ethanol umkristallisiert.
Farblose Kristalle, F. 83° C, Ausbeute: 64 %

Beispiel 10

1-[4-(N,N-Bis-β-chlorethylcarbamoyloxy)-phenyl]-1-(4-acetoxyphenyl)-2-phenyl-but-1-en

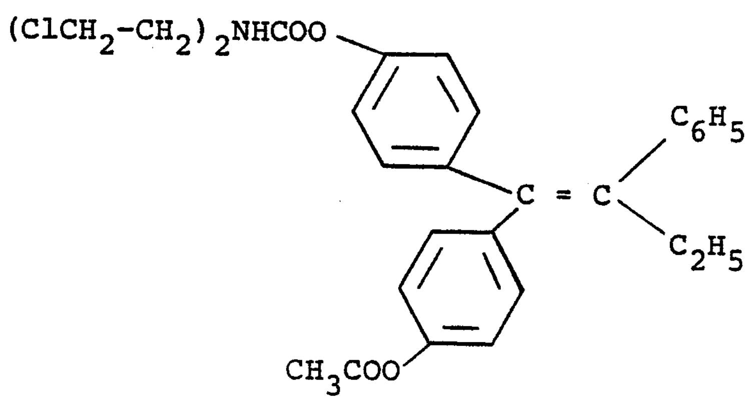

Die Herstellung erfolgt aus 1-[4-(N,N-Bis-β-chlorethylcarbamoyloxy)-phenyl]-1-(4-hydroxy-phenyl)-2-phenyl-but-1-en und Acetanhydrid analog Beispiel 7.
F. 58° C, Ausbeute: 85 %

Beispiel 11

1-[4-(N,N-Bis-β-chlorethylcarbamoyloxy)-phenyl]-1-(4-acetoxyphenyl)-2-(3-acetoxyphenyl)-but-1-en

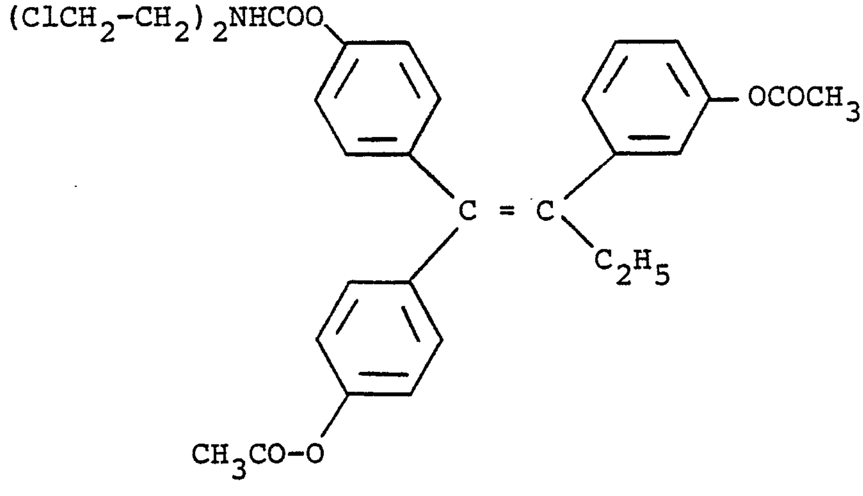

Die Herstellung erfolgt aus 1-[4-(N,N-Bis-β-chlorethylcarbamoyloxy)-phenyl]-1-(4-hydroxyphenyl)-2-(3-hydroxyphenyl)-but-1-en und Acetanhydrid analog Beispiel 7

Da die Substanz keinen Schmelzpunkt hat, wird sie durch das kernmagnetische Resonanzspektrum $^1H$-NMR charakterisiert.

Analysenwerte:
$C_{31}H_{31}Cl_2NO_6$ (584,5)
Ber.: C 63, 69 H 5,36 N 2,40
Gef.: C 63,30 H 5,32 N 2,40
$^1H$-NMR Aufnahme in deuteriertem Chloroform bei 90 Megahertz
= 1,00 (t,J=8Hz, 3H,$CH_3$); 2,40 (t,6H,$COCH_3$); 2,50 (q,J=8Hz,2H,$CH_2$); 3,85 (d,8H,$N(CH_2CH_2Cl)_2$); 7,00-7,44 - (m,12H,aromat.H)

Beispiel 12

1,1-Bis-(4-monochloracetoxyphenyl)-2-phenyl-but-1-en

1,6 g (5 mmol) 1,1-Bis-(4-hydroxyphenyl)-2-phenyl-but-1-en werden in 5,7 g (50 mmol) Chloracetylchlorid suspendiert und 1 Stunde bei 120° C gerührt. Das überschüssige Chloracetylchlorid wird abdestilliert und das Rohprodukt über eine Kieselgelsäule (Laufmittel: $CHCl_3$/Essigester 10:1) gereinigt. Das ölige Produkt kristallisiert in Ethanol.
Farblose Kristalle, F. 115° C, Ausbeute: 68 %.

Beispiel 13

1,1-Bis-(4-monobromacetoxyphenyl)-2-phenyl-but-1-en

Herstellung erfolgt analog Beispiel 12 unter Verwendung von 50 mmol Bromacetylchlorid.
Farblose Kristalle, F. 85° C, Ausbeute: 64 %.

Beispiele für pharmazeutische Zubereitungen

Kapseln 10 mg Wirkstoff

10 g Wirkstoff nach Beispiel 1 werden mikronisiert, mit 106,7 g Calciumhydrogenphosphat durch ein Sieb (1 mm Maschenweite) gegeben und homogenisiert. Diese Mischung wird mit einer Lösung aus 2,3 g Gelatine und 1,0 g Polyoxyethylen(20)sorbitanmonooleat in 20,7 g Wasser angefeuchtet, durch ein Sieb der Maschenweite 2 mm granuliert und bei 40° C getrocknet. Das trockene Granulat und 20 g Maisstärke passiert man durch ein 0,8 mm-Sieb und homogenisiert. Diese Masse wird auf einer geeigneten Kapselmaschine zu 140 mg in Hartgelatinesteckkapseln der Größe 3 abgefüllt. 1 Kapsel enthält 10 mg Wirkstoff.

Tabletten 10 mg Wirkstoff

10 g Wirkstoff nach Beispiel 3 (mikronisiert) und 107,6 g Calciumhydrogenphosphat werden gemischt und mit einer Lösung aus 2,3 g Gelatine und 0,1 g Polyoxyethylen(20)sorbitanmonooleat in 20,6 g Wasser angefeuchtet. Diese Masse wird durch ein 2 mm-Sieb granuliert und bei 40° C getrocknet. Anschließend gibt man dieses Granulat, 35 g Maisstärke und 5 g Talkum durch ein Sieb (0,8 mm Maschenweite) und homogenisiert. In üblicher Weise wird diese Masse auf einer geeigneten Maschine zu Tabletten von 160 mg Gewicht und einem Durchmesser von 7 mm gepreßt. 1 Tablette enthält 10 mg Wirkstoff.

Injektionslösung

5 g Wirkstoff nach Beispiel 6 werden unter Rühren und Erwärmen auf ca 40° C in 1,8 Liter Erdnußöl für Injektionszwecke gelöst und auf 2 Liter aufgefüllt. Unter aseptischen Bedingungen wird diese Injektionslösung durch Filtration über keimdichte Filter (0,2 µm Porenweite) sterilisiert.
Schließlich füllt man unter aseptischen Bedingungen und unter Stickstoffbegasung 1,1 ml (Nennvolumen 1 ml) in sterile 2 ml Ampullen ab. 1 Ampulle enthält 2,5 mg Wirkstoff.

## Ansprüche

1. Substituierte 1,1,2-Triphenylbut-1-ene der allgemeinen Formel

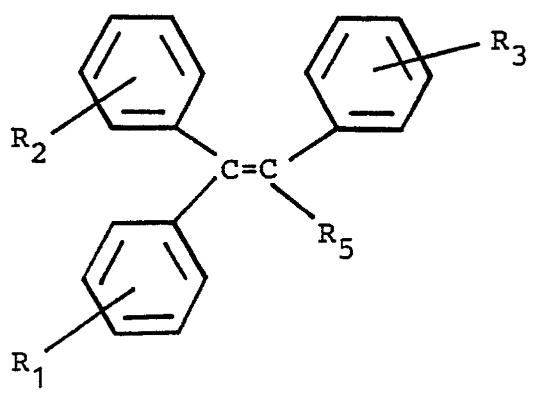

I

worin die Reste $R_1$, $R_2$ und $R_3$ gleich oder verschieden sind und Wasserstoff oder die Gruppe $OR_4$ bedeuten, und $R_4$ eine $C_3$-$C_6$-Alkenoylgruppe, eine Carbamoylgruppe, eine Carbamoylgruppe, die einen oder zwei $\beta$-Chlorethylreste oder $\beta$-Bromethylreste enthält, oder eine $C_2$-$C_6$-Alkanoylgruppe, welche ein Halogenatom enthält, bedeutet und $R_5$ eine $C_1$-$C_6$-Alkylgruppe darstellt, wobei mindestens einer der Reste $R_1$, $R_2$ oder $R_3$ kein Wasserstoffatom ist, und worin einer oder zwei der Reste $R_1$, $R_2$ und $R_3$ auch eine Hydroxygruppe, die Gruppe $-OPO(OH)_2$, eine $C_2$-$C_6$-Alkanoyloxygruppe, welche gegebenenfalls eine Carboxygruppe enthält, oder eine 2-(Di-$C_1$-$C_4$-alkylamino)-ethyloxygruppe sein können, falls einer oder zwei dieser Reste die Carbamoyloxygruppe, oder eine Carbamoyloxygruppe, die einen oder zwei β-Chlorethylreste oder β-Bromethylreste enthält, darstellen und deren Salze mit therapeutisch verträglichen Kationen.

2. Verbindungen nach Anspruch 1,
worin $R_2$ eine Carbamoyloxygruppe ist, die einen oder zwei β-Chlorethyl-oder β-Bromethylreste enthält und $R_1$ eine Hydroxygruppe, eine $C_3$-$C_6$-Alkenoyloxygruppe, eine Carbamoyloxygruppe, die Gruppe -OPO(OH)$_2$ oder eine $C_2$-$C_6$-Alkanoyloxygruppe, welche gegebenenfalls eine Carboxygruppe oder ein Halogenatom enthält, bedeutet, falls $R_3$ Wasserstoff oder eine Hydroxygruppe darstellt, oder worin $R_3$ eine Hydroxygruppe, eine $C_3$-$C_6$-Alkenoyloxygruppe, eine Carbamoyloxygruppe, die Gruppe -OPO(OH)$_2$ oder eine $C_2$-$C_6$-Alkanoyloxygruppe, welche gegebenenfalls eine Carboxygruppe oder ein Halogenatom enthält, bedeutet, falls $R_1$ Wasserstoff oder eine Hydroxygruppe darstellt, und deren Salze mit therapeutisch verträglichen Kationen.

3. Verfahren zur Herstellung von substituierten 1,1,2-Triphenylbut-1-enen der allgemeinen Formel

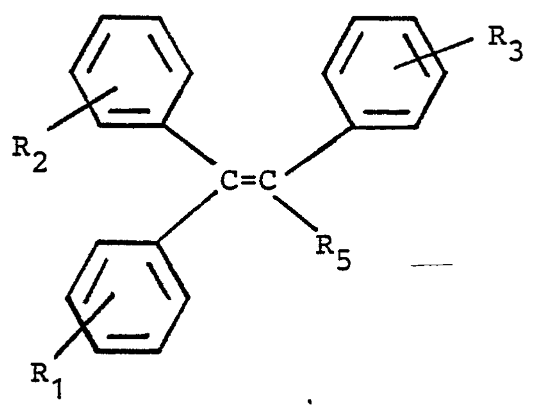

I

worin die Reste $R_1$, $R_2$ und $R_3$ gleich oder verschieden sind und Wasserstoff oder die Gruppe $OR_4$ bedeuten, und $R_4$ eine $C_3$-$C_6$-Alkenoylgruppe, eine Carbamoylgruppe, eine Carbamoylgruppe, die einen oder zwei β-Chlorethylreste oder β-Bromethylreste enthält, oder eine $C_2$-$C_6$-Alkanoylgruppe, welche ein Halogenatom enthält, bedeutet und $R_5$ eine $C_1$-$C_6$-Alkylgruppe darstellt, wobei mindestens einer der Reste $R_1$, $R_2$ oder $R_3$ kein Wasserstoffatom ist, und worin einer oder zwei der Reste $R_1$, $R_2$ und $R_3$ auch eine Hydroxygruppe, die Gruppe -OPO(OH)$_2$, eine $C_2$-$C_6$-Alkanoyloxygruppe, welche gegebenenfalls eine Carboxygruppe enthält, oder eine 2-(Di-$C_1$-$C_4$-alkylamino)-ethyloxygruppe sein können, falls einer oder zwei dieser Reste die Carbamoyloxygruppe, oder eine Carbamoyloxygruppe, die einen oder zwei β-Chlorethylreste oder β-Bromethylreste enthält, darstellen und deren Salze mit therapeutisch verträglichen Kationen,
dadurch gekennzeichnet,
daß man in eine Verbindung der Formel

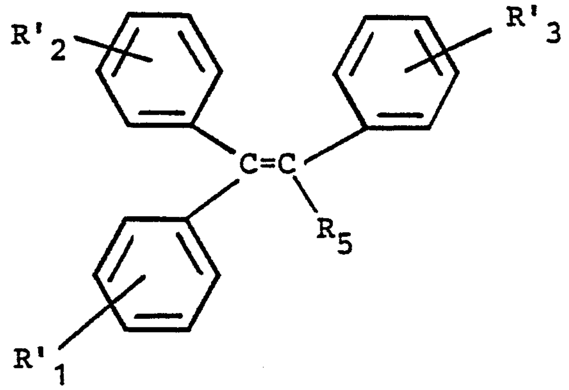

II

worin $R_5$ die angegebenen Bedeutungen hat und die Reste $R'_1$, $R'_2$ und $R'_3$ Hydroxygruppen bedeuten, oder einer oder zwei der Reste $R'_1$, $R'_2$, $R'_3$ auch Wasserstoff oder eine $C_1$-$C_6$-Alkoxygruppe sein können, durch Acylierung der Hydroxygruppe(n) den Rest $R_4$ einführt, wobei $R_4$ die angegebenen Bedeutungen außer Wasserstoff hat, anschließend gegebenenfalls vorhandene $C_1$-$C_6$-Alkoxygruppen abspaltet, in so erhaltene Hydroxygruppen gegebenenfalls $R_4$ einführt, gegebenenfalls aus einer $C_2$-$C_6$-Alkanoylgruppe, die ein Halogenatom enthält, Halogenwasserstoff abspaltet und erhaltene Verbindungen mit einer Carboxygruppe gegebenenfalls in ihre Salze überführt.

4. Verbindungen der Formel I gemäß Anspruch 1 oder Anspruch 2 zur Anwendung als therapeutische Wirkstoffe.

5. Arzneimittel, enthaltend eine Verbindung der allgemeinen Formel I gemäß Anspruch 1 oder Anspruch 2 neben üblichen Träger-und/oder Verdünnungs-beziehungsweise Hilfsstoffen.

6. Verfahren zur Herstellung eines Arzneimittels,
dadurch gekennzeichnet,
daß eine Verbindung der allgemeinen Formel I gemäß Anspruch 1 oder Anspruch 2 mit gebräuchlichen pharmazeutischen Trägerstoffen und/oder Verdünnungsmitteln beziehungsweise sonstigen Hilfsstoffen zu pharmazeutischen Zubereitungen verarbeitet beziehungsweise in eine therapeutisch anwendbare Form gebracht wird.

7. Verwendung von Verbindungen der allgemeinen Formel I gemäß Anspruch 1 oder Anspruch 2 zur Herstellung von Arzneimitteln.

Patentansprüche für folgende Vertragsstaten : AT; ES; GR.

1. Verfahren zur Herstellung von substituierten 1,1,2-Triphenylbut-1-enen der allgemeinen Formel

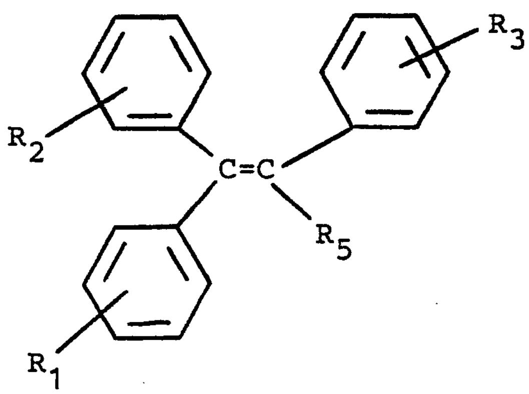

I

worin die Reste $R_1$, $R_2$ und $R_3$ gleich oder verschieden sind und Wasserstoff oder die Gruppe $OR_4$ bedeuten, und $R_4$ eine $C_3$-$C_6$-Alkenoylgruppe, eine Carbamoylgruppe, eine Carbamoylgruppe, die einen oder zwei $\beta$-Chlorethylreste oder $\beta$-Bromethylreste enthält, oder eine $C_2$-$C_6$-Alkanoylgruppe, welche ein Halogenatom enthält, bedeutet und $R_5$ eine $C_1$-$C_6$-Alkylgruppe darstellt, wobei mindestens einer der Reste $R_1$, $R_2$ oder $R_3$ kein Wasserstoffatom ist, und worin einer oder zwei der Reste $R_1$, $R_2$ und $R_3$ auch eine Hydroxygruppe, die Gruppe $-OPO(OH)_2$, eine $C_2$-$C_6$-Alkanoyloxygruppe, welche gegebenenfalls eine Carboxygruppe enthält, oder eine 2-(Di-$C_1$-$C_4$-alkylamino)-ethyloxygruppe sein können, falls einer oder zwei dieser Reste die Carbamoyloxygruppe, oder eine Carbamoyloxygruppe, die einen oder zwei $\beta$-Chlorethylreste oder $\beta$-Bromethylreste enthält, darstellen und deren Salze mit therapeutisch verträglichen Kationen
dadurch gekennzeichnet,
daß man in eine Verbindung der Formel

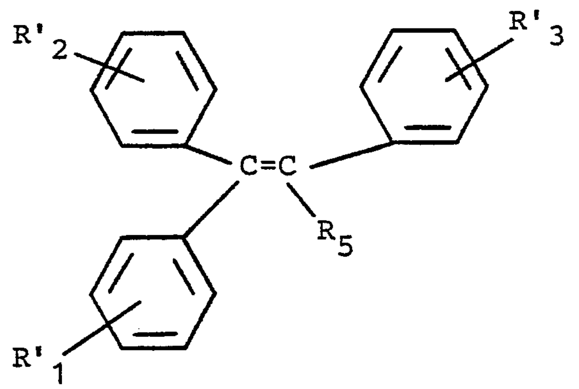

II

worin $R_5$ die angegebenen Bedeutungen hat und die Reste $R'_1$, $R'_2$ und $R'_3$ Hydroxygruppen bedeuten, oder einer oder zwei der Reste $R'_1$, $R'_2$, $R'_3$ auch Wasserstoff oder eine $C_1$-$C_6$-Alkoxygruppe sein können, durch Acylierung der Hydroxygruppe(n) den Rest $R_4$ einführt, wobei $R_4$ die angegebenen Bedeutungen außer Wasserstoff hat, anschließend gegebenenfalls vorhandene $C_1$-$C_6$-Alkoxygruppen abspaltet, in so erhaltene Hydroxygruppen gegebenenfalls $R_4$ einführt und erhaltene Verbindungen mit einer Carboxygruppe gegebenenfalls in ihre Salze überführt.

2. Verfahren zur Herstellung eines Arzneimittels, enthaltend als Wirkstoff mindestens eine Verbindung der allgemeinen Formel I, worin die Reste $R_1$, $R_2$, $R_3$, $R_4$ und $R_5$ die in Anspruch 1 angegebene Bedeutung haben,
dadurch gekennzeichnet,
daß mindestens eine Verbindung der Formel I mit gebräuchlichen pharmazeutischen Trägerstoffen und/oder Verdünnungsmitteln beziehungsweise sonstigen Hilfsstoffen zu pharmazeutischen Zubereitungen verarbeitet beziehungsweise in eine therapeutisch anwendbare Form gebracht wird.

3. Verfahren zur Herstellung eines (antitumorwirksamen) Arzneimittels,
dadurch gekennzeichnet
daß man Verbindungen der Formel I, worin die Reste $R_1$, $R_2$, $R_3$, $R_4$ und $R_5$ die in Anspruch 1 angegebene Bedeutung haben, beziehungsweise deren Salze mit physiologisch unbedenklichen Säuren zusammen mit üblichen Träger-und/oder Verdünnungs-beziehungsweise Hilfsstoffen bei Temperaturen zwischen 20 und 100° C vermischt beziehungsweise homogenisiert, die so erhaltene Mischung zur Herstellung von Zubereitungen, die in der Dosierungseinheit 10 bis 200 mg Wirkstoff der Formel I enthalten, in Hohlzellen entsprechender Größe ausgießt oder in Kapseln entsprechender Größe abfüllt oder granuliert und dann gegebenenfalls unter Zusatz von weiteren üblichen Hilfsstoffen zu Tabletten verpreßt.

4. Verfahren zur Herstellung eines (antitumorwirksamen) Arzneimittels,
dadurch gekennzeichnet
daß man Verbindungen der Formel I, worin die Reste $R_1$, $R_2$, $R_3$, $R_4$ und $R_5$ die in Anspruch 1 angegebene Bedeutung haben, beziehungsweise deren Salze mit physiologisch unbedenklichen Säuren mit einem oder mehreren der folgenden Stoffe: Stärke, Cellulose, Lactose, Formalin-Casein, modifizierte Stärke, Magnesiumstearat, Calciumhydrogenphosphat, hochdisperse Kieselsäure, Talkum vermischt, die erhaltene Mischung, gegebenenfalls mit einer wässrigen Lösung, die als Bestandteil mindestens Gelatine, Stärke, Vinylpyrrolidon-Vinylacetat-Copolymerisat und/oder Polyoxyethylensorbitanmonooleat enthält, granuliert, das Granulat gegebenenfalls mit einem oder mehreren der obengenannten Hilfsstoffe homogenisiert, und diese Mischung zu Tabletten verpresst, die in der Dosierungseinheit 10 bis 200 mg Wirkstoff der Formel I enthalten.

5. Verfahren zur Herstellung eines (antitumorwirksamen) Arzneimittels,
dadurch gekennzeichnet,
daß man Verbindungen der Formel I oder deren Salze mit physiologisch unbedenklichen Säuren nach Zusatz von Sojalecithin bei Temperaturen zwischen 33 -37° C in geschmolzenem Hartfett suspendiert und homogenisiert und anschließend die Mischung in Hohlzellen ausgießt, wobei die Dosierungseinheit 10 bis 200 mg Wirkstoff enthält.

6. Verfahren zur Herstellung einer antitumorwirksamen Injektionslösung,
dadurch gekennzeichnet,
daß man Verbindungen der Formel I oder deren Salze mit physiologisch unbedenklichen Säuren in Pflanzenöl bei Temperaturen zwischen 30 -100° C auflöst, und die so erhaltene Lösung mit soviel Pflanzenöl auffüllt, daß die Endlösung 0,01 bis 1 Gewichtsprozent an Wirkstoff der Formel I enthält.

7. Verwendung von Verbindungen der allgemeinen Formel I, worin die Reste $R_1$, $R_2$, $R_3$, $R_4$ und $R_5$ die in Anspruch 1 angegebene Bedeutung haben, zur Herstellung von Arzneimitteln.